# EUROPEAN PATENT APPLICATION

(11) **EP 4 480 581 A1**
(43) Date of publication of application: **25.12.2024**
(21) Application number: 23182831.0
(22) Date of filing: 30.06.2023
(51) Int. Cl.: B01L 3/00

(54) **FLUIDIC DEVICE AND USE OF THE SAME**

(30) Priority: 23.06.2023 EP 23181319
(71) Applicant: Koninklijke Philips N.V., 5656 AG Eindhoven (NL)
(72) Inventor: BECKERS, Lucas Johannes Anna Maria, 5656AG Eindhoven (NL); BERBEN, Edward Theodorus Maria, 5656AG Eindhoven (NL); LIPSCH, Job, 5656AG Eindhoven (NL); VALSTER, Susanne Maaike, 5656AG Eindhoven (NL)
(74) Representative: Philips Intellectual Property & Standards

(57) **Abstract**

Provided is a fluidic device (100) for testing biological material. The fluidic device comprises a substrate (102) having a top side (104) and a bottom side (106). The fluidic device further comprises one or more fluidic assemblies (108). Each of the one or more fluidic assemblies comprises at least one chamber unit (110) in which biological material is receivable. The at least one chamber unit is defined in the substrate and extends between the top side and the bottom side. Each of the one or more fluidic assemblies also comprises an inlet (112), an outlet (114), an inlet fluid channel (116) defined in the substrate and extending between the inlet and the at least one chamber unit, and an outlet fluid channel (118) defined in the substrate and extending between the at least one chamber unit and the outlet.

## Description

### FIELD OF THE INVENTION

The invention relates to a fluidic device for testing biological material.

The invention also relates to use of such a fluidic device for testing biological material.

The invention further relates to a drug testing method in which the fluidic device is used.

### BACKGROUND OF THE INVENTION

Fluidic devices can be used in a number of different applications, such as for cell or tissue culturing, for example for biopsy study. So-called organ-on-chip devices use microfluidics along with cells to imitate the physiological and mechanical conditions experienced in the body. Such organ-on-chip devices have found utility in, for instance, oncology.

Fluidic devices can include two or more fluid channels for carrying fluids to a culturing chamber. One channel can be used, for example, to provide nutrients and oxygen to the culturing chamber, as well as to remove metabolic products such as carbon dioxide therefrom. A further channel can be used to provide medication to the culturing chamber. In such an example, the channels can be arranged at different depths of the fluidic device, such that one of the channels is positioned on top of, albeit separated from, the other channel.

Various challenges remain in terms of controlling flow of fluids in such fluid channels, particularly when the fluidic device comprises more than one culturing chamber, such as when the fluidic device comprises a substrate in which an array of culturing chambers is defined.

### SUMMARY OF THE INVENTION

The invention is defined by the claims.

According to examples in accordance with an aspect of the invention, there is provided a fluidic device for testing biological material, the fluidic device comprising: a substrate having a top side and a bottom side; and one or more fluidic assemblies that each comprise: at least one chamber unit in which the biological material is receivable, the at least one chamber unit being defined in the substrate and extending between the top side and the bottom side; an inlet for receiving fluid; an outlet for allowing removal of fluid; an inlet fluid channel defined in the substrate and extending between the inlet and the at least one chamber unit; an outlet fluid channel defined in the substrate and extending between the at least one chamber unit and the outlet; and a dam assembly for controlling fluid flow through the at least one chamber unit, the dam assembly comprising at least one of an inlet dam and an outlet dam, the inlet dam being between the inlet fluid channel and the at least one chamber unit and upstanding towards the top side to an inlet threshold arranged to permit fluid from the inlet fluid channel to flow thereover to be received in the at least one chamber unit, and the outlet dam being between the at least one chamber unit and the outlet fluid channel and upstanding towards the top side to an outlet threshold arranged to permit fluid from the at least one chamber unit to flow thereover to be received in the outlet fluid channel.

Fluid flow through the at least one chamber unit may be better controlled due to inclusion of the dam assembly. It is noted that the dam assembly may be used to control the fluid flow when the fluidic device is orientated for use, for example with the substrate's plane, e.g. a plane of the bottom side of the substrate, being horizontal.

In embodiments in which the dam assembly comprises the inlet dam upstanding between the inlet fluid channel and the at least one chamber unit, the inlet fluid channel may be configured to fill up to the inlet threshold prior to the fluid flowing over the inlet threshold to enter the chamber unit(s).

In embodiments in which several chamber units are each separated from the inlet fluid channel by a respective inlet dam, the inlet thresholds of at least some of the inlet dams may be at different heights relative to each other.

In such embodiments, stepwise filling of the chamber units, e.g. one by one, may be facilitated. This may obviate tilting of the substrate, e.g. by 5 to 10 degrees from the horizontal, in order to implement such stepwise filling of the chamber units.

In embodiments in which the dam assembly comprises the outlet dam upstanding between the at least one chamber unit and the outlet fluid channel, the outlet dam can assist to collect sufficient fluid, e.g. cell culture medium, over the biological material. For instance, by shaping, e.g. selecting the height of, the outlet threshold, larger fluid reservoir(s) can be provided.

In embodiments in which the fluidic device comprises a plurality of the fluidic assemblies, the outlet thresholds of at least some of the plurality of fluidic assemblies may be at different heights relative to each other. Such a fluidic device may enable testing to identify a suitable height of the outlet threshold, e.g. as part of process to optimize cell culturing conditions.

In embodiments in which the fluidic device comprises a plurality of chamber units supplied with fluid by the inlet fluid channel and arranged so that fluid is removed from the chamber units by the outlet fluid channel, the dam assembly, e.g. comprising the first and second dams, may allow emptying of one or more of such chambers, e.g. by pipetting, without disturbing fluid levels in other chamber(s).

In some embodiments, the at least one chamber unit extends to an opening at the top side of the substrate, and the dam assembly comprises the inlet dam and the outlet dam, with the inlet dam and the outlet dam being arranged to retain fluid in the at least one chamber unit.

In such embodiments, the fluidic device may be operable as a so-called open system in which fluid delivery into and/or fluid withdrawal from the at least one chamber unit via its opening can be implemented, e.g. using a pipette arrangement, without having to connect a flow control system to the inlet and the outlet. This capability is due to the chamber unit(s) retaining fluid therein irrespective of whether or not there is flow of fluid being provided via the inlet fluid channel.

In some embodiments, the outlet threshold of the outlet dam is lower than the inlet threshold of the inlet dam so that fluid filling the at least one chamber unit to reach the outlet threshold flows over the outlet threshold and into the outlet fluid channel.

This arrangement enables the fluidic device to be additionally utilizable as a closed system in which the flow control system drives flow in the inlet fluid channel and the outlet fluid channel. By the outlet threshold being lower than the inlet threshold, the risk of undesirable backflow from the chamber unit(s) into the inlet fluid channel may be minimized.

In some embodiments, a dual chamber unit is included in one or more, e.g. all, of the at least one chamber unit, with the dual chamber unit comprising a first chamber and a second chamber fluidly connected to each other via a bridge defined between the first chamber and the second chamber. Such a dual chamber unit may have utility in studying metastasis of cancerous cells. It may be possible to assess cells that are released or emitted by any cell cultures that are provided in the first chamber. The first chamber can therefore be used to perform a treatment on the cell culture(s), such as the application of a certain therapeutic fluid or drug, for example a chemotherapy treatment fluid, with the second chamber capturing any released cells to analyze the effect of the treatment performed in the first chamber on metastasis.

In such embodiments, the inlet dam may be between the inlet fluid channel and the first chamber so that fluid filling the inlet fluid channel to reach the inlet threshold flows over the inlet threshold into the first chamber. Moreover, the outlet dam may be between the second chamber and the outlet fluid channel.

The fluidic device, and more particularly each of the one or more fluidic assemblies, may further comprise an intermediate dam between the first and second chambers, with the intermediate dam upstanding towards the top side of the substrate to a bridge threshold at a base of the bridge between the first chamber and the second chamber.

In such embodiments, the bridge threshold is preferably lower than the inlet threshold so that fluid filling the first chamber to reach the bridge threshold is permitted to flow over the bridge threshold into the second chamber.

Moreover, the outlet threshold is preferably lower than the bridge threshold so that fluid filling the second chamber to reach the outlet threshold flows over the outlet threshold into the outlet fluid channel.

This ordering of heights of the inlet threshold, the bridge threshold and the outlet threshold may assist to minimize the risk of undesirable backflow during closed system-type operation of the fluidic device using the fluid control system. It is also reiterated that removing fluid, e.g. old fluid, from and adding fresh fluid to the chamber unit(s) can also be achieved via pipetting.

In at least some embodiments, each of the at least one chamber unit comprises at least one supporting region for supporting a biocompatible partition so that the biocompatible partition separates an upper portion from a lower portion of the respective chamber unit. The biological material may be supported by the biocompatible partition when the biological material is received in the chamber unit(s).

It is noted that the inlet dam and/or the outlet dam may at least partly delimit the upper portion.

In some embodiments, the supporting region(s) comprise(s) a support ledge, provided around an inner perimeter of the respective chamber unit, on which the biocompatible partition can be supported.

In at least some embodiments, the biocompatible partition is suitable for culturing cells thereon. Such a biocompatible partition is preferably in the form of a perforated membrane or film.

The biocompatible partition may be attachable to the substrate. In such embodiments, the biocompatible partition and the substrate may be separately manufacturable, for example from different materials.

In embodiments in which the substrate is formed from the same material(s) as the biocompatible partition, the fabrication process may comprise forming the substrate and integral biocompatible partition, for example in a molding, e.g. injection molding, process.

In such embodiments, the fabrication process may further comprise forming apertures in the integral biocompatible partition, e.g. via laser ablation.

In some embodiments, the upper and lower portions separated by the supporting region(s) have different volumes relative to each other, for example with the lower portion having a smaller volume than that of the upper portion. This may be beneficial when testing a drug formulation that is more appropriately supplied, e.g. for cost and/or scarcity of supply reasons, to whichever of the upper and lower portions has the smaller volume.

Alternatively or additionally, the at least one supporting region may be arranged to support the biocompatible partition closer to one of the top and bottom sides than the other of the top and bottom sides. In some embodiments, the supporting region(s) is or are configured to support the biocompatible partition closer to the bottom side than to the top side. This may facilitate monitoring of the biological material received in the chamber unit(s), particularly by optical microscopy.

The fluidic device, and more particularly each of the one or more fluidic assemblies, may comprise an upper fluid flow system defined in the substrate and arranged to supply fluid and permit fluid to leave the upper portion of the chamber unit(s).

Each of the one or more fluidic assemblies may also comprise a lower fluid flow system defined in the substrate and arranged to supply fluid to and/or permit fluid to leave the lower portion of the chamber unit(s).

The upper and lower fluid flow systems may fulfil different functions relative to each other, for example by the upper fluid flow system supplying cell nourishment to the upper portion of the chamber unit(s), while the lower fluid flow system supplies a drug formulation to the lower portion of the chamber unit(s).

To this end, the upper and lower fluid flow systems may be mutually independently controlled in respect of flow and/or composition of fluid to create various, e.g. the same or different, conditions in the upper portion and the lower portion of the chamber unit(s).

In embodiments in which each of the one or more fluidic assemblies includes a plurality of chamber units, the chamber units may be connected in parallel across the inlet fluid channel and the outlet fluid channel. Such a design can facilitate efficient connection to the flow control system due to fewer connection tubing points being required, whilst also assisting to minimize cross-contamination between chamber units.

In embodiments in which the plurality of chamber units each comprise the upper portion and the lower portion, the lower fluid flow system may comprise at least one lower fluid channel that fluidly connects the lower portions of the chamber units to each other.

In embodiments in which the fluidic device comprises a plurality of fluidic assemblies that each include chamber units connected in parallel across the inlet fluid channel and the outlet fluid channel, the lower fluid flow system may include lower fluid channels that each provide a fluid connection between lower portions of chamber units belonging to different fluidic assemblies.

This may reduce the risk of blockages in the fluidic device.

In some embodiments, the inlet fluid channel provides a resistance to flow therein that changes with distance along the inlet fluid channel away from the inlet, for example by variation of the dimensions, e.g. cross-sectional area, of the inlet fluid channel with distance along the inlet fluid channel away from the inlet.

In such embodiments, a narrower portion of the inlet fluid channel may be provided proximal to the inlet, followed by a wider portion of the inlet fluid channel along the inlet fluid channel further away from the inlet.

In embodiments in which the chamber units are connected in parallel across the inlet fluid channel and the outlet fluid channel, the resistance to flow in the inlet fluid channel may decrease, for example due to the inlet fluid channel having increased cross-sectional area, between fluid inlets, e.g. inlet thresholds, of successive chamber units in a downstream direction away from the inlet.

Such a design of the inlet fluid channel may assist to control, e.g. provide equal continuous flow, across each of the parallel chamber units.

Alternatively or additionally, the outlet fluid channel may provide a resistance to flow therein that changes with distance along the outlet fluid channel away from the fluid outlets, e.g. outlet thresholds, of the chamber unit(s), for example by variation of the dimensions, e.g. cross-sectional area, of the outlet fluid channel with distance along the outlet fluid channel away from the fluid outlets.

In some embodiments, the fluidic device comprises an upper cover for covering the top side of the substrate and/or a lower cover for covering the bottom side of the substrate.

Such a cover or covers may assist retention of fluid in and/or contamination protection of the at least one chamber unit. Regarding fluid retention, such an upper cover and/or lower cover can assist to minimize loss of fluid via evaporation.

In some embodiments, one or both of the upper and lower covers is or are configured to enable access to the at least one chamber unit, for example for fluid delivery into and/or fluid withdrawal from the at least one chamber unit, e.g. via the above-mentioned pipette arrangement.

For example, one or both of the upper and lower covers may be releasably mounted, e.g. detachably mounted, to the substrate, with release, e.g. detachment, of the respective cover enabling access to the at least one chamber unit.

The upper and lower covers can be formed of any suitable material. Particular mention is made of optically transmissive materials, such as glass and optically transmissive polymers, such as polycarbonate. This is because such materials may facilitate monitoring of testing taking place in the fluidic device, for instance via optical microscopy.

According to another aspect there is provided a use of the fluidic device according to any one of the embodiments described herein for testing biological material.

In some embodiments, the biological material being tested comprises cell groups, double cell layers, spheroids, organoids and/or biopsies.

According to a further aspect there is provided a drug testing method comprising: providing biological material in the at least one chamber unit of a fluidic device according to any of the embodiments described herein; and exposing the biological material to the drug to be tested.

In some embodiments, exposing the biological material to the drug to be tested comprises delivering the drug to be tested using one or both of the upper and lower fluid flow system(s).

Alternatively or additionally, the providing of biological material in the at least one chamber unit may comprise culturing cells in the at least one chamber unit.

Alternatively or additionally, the method may comprise monitoring a response of the biological material, e.g cultured cells, to the drug to be tested. This monitoring may be implemented in any suitable manner, for example by optical microscopy.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
FIGs. 1A and 1B provide views of a fluidic device according to a first example;
FIG. 2 provides a schematic cross-sectional view of part of a fluidic device according to a second example;
FIG. 3 provides a cutaway view of a fluidic device according to a third example;
FIGs. 4 and 5 provide cutaway views of a fluidic device according to a fourth example;
FIGs. 6A to 6E provide various views of a fluidic device according to a fifth example;
FIGs. 7A and 7B provide views of a fluidic device according to a sixth example;
FIG. 8 provides a perspective view of a top side of a fluidic device according to a seventh example;
FIGs. 9A and 9B respectively provide perspective views of a top side and a bottom side of a fluidic device according to an eighth example; and
FIGs. 10A and 10B respectively provide perspective views of a top side and a bottom side of a fluidic device according to a ninth example.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Provided is a fluidic device for testing biological material. The fluidic device comprises a substrate having a top side and a bottom side. The fluidic device further comprises one or more fluidic assemblies. Each of the one or more fluidic assemblies comprises at least one chamber unit in which biological material is receivable. The at least one chamber unit is defined in the substrate and extends between the top side and the bottom side. Each of the one or more fluidic assemblies also comprises an inlet, an outlet, an inlet fluid channel defined in the substrate and extending between the inlet and the at least one chamber unit, and an outlet fluid channel defined in the substrate and extending between the at least one chamber unit and the outlet.

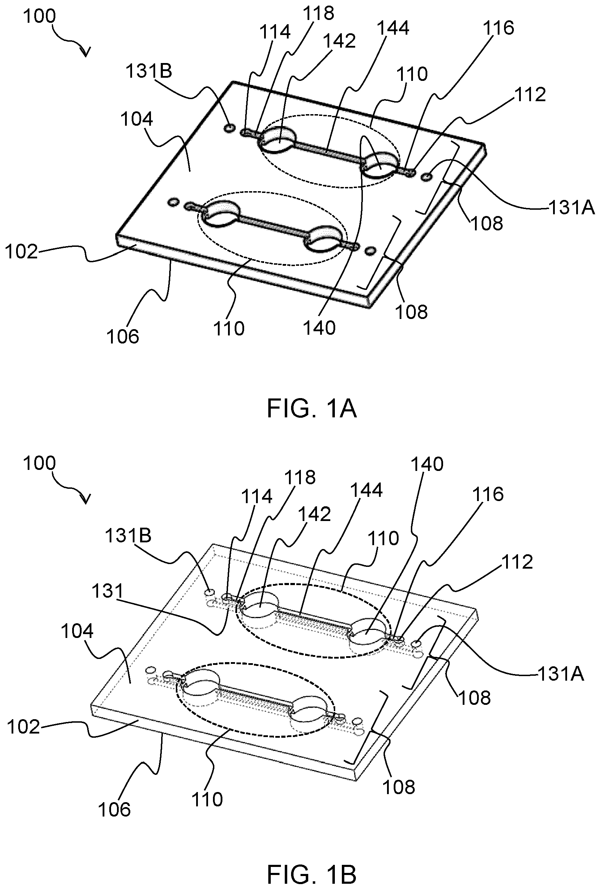

FIGs. 1A and 1B provide views of a fluidic device 100. The fluidic device 100 comprises a substrate 102 having a top side 104 and a bottom side 106. The fluidic device 100 comprises one or more fluidic assemblies 108, and in the case of the embodiment shown in FIGs. 1A and 1B the fluidic device 100 comprises two fluidic assemblies 108. The number of fluidic assemblies 108 included in the fluidic device 100 can vary according to testing requirements, and in other embodiments the fluidic device 100 comprises a single fluidic assembly 108 or three, four, five, six, seven, eight or more fluidic assemblies 108.

Each of the one or more fluidic assemblies 108 comprises at least one chamber unit 110 in which biological material is receivable. The at least one chamber unit 110 is defined in the substrate 102 and extends between the top side 104 and the bottom side 106.

The at least one chamber unit 110 may extend to an upper opening delimited by the top side 104 of the substrate 102 and/or may extend to a lower opening delimited by the bottom side 106 of the substrate 102. In such embodiments, fluid retention in and/or contamination protection of the at least one chamber unit 110 can be assisted by the fluidic device 100 including an upper cover (not visible) for covering the top side 104 of the substrate 102 and/or a lower cover (not visible) for covering the bottom side 106 of the substrate 102. Regarding fluid retention, such an upper cover and/or lower cover can assist to minimize loss of fluid via evaporation.

In some embodiments, one or both of the upper and lower covers is or are configured to enable access to the at least one chamber unit 110, for example for fluid delivery into and/or fluid withdrawal from the at least one chamber unit 110, e.g. via a pipette arrangement.

For example, one or both of the upper and lower covers may be releasably mounted to the substrate 102, with release of the respective cover enabling access to the at least one chamber unit 110.

The upper and lower covers can be formed of any suitable material. Particular mention is made of optically transmissive materials, such as glass and optically transmissive polymers, such as polycarbonate. This is because such materials may facilitate monitoring of testing taking place in the fluidic device 100, for instance via optical microscopy.

The upper and lower covers can be mounted, e.g. releasably mounted, to the substrate 102 in any suitable manner, for example using adhesive tape, clips, and so on. A non-limiting example of suitable adhesive tape is Avery Dennison MED 1125.

More generally, biological material is receivable in the chamber unit(s) 110 and testing of the biological material may be carried out using the fluidic device 100. Examples of such biological material include cell groups, double cell layers, spheroids, organoids or biopsies.

Such biological material tends to be required to be living during at least part of the testing performed using the fluidic device 100. To this end, the fluidic device 100, for example the substrate 102 thereof, may be formed from a suitable biocompatible material. In some embodiments, the substrate 102 is formed from a biocompatible polymeric material, such as silicone, e.g. to which a suitable cell culture protein, such as fibronectin, is applied.

In order to test the biological material received in the chamber unit(s) 110, the fluidic assembly 108 includes an inlet 112 at which fluid, for example fluid containing cell nourishment and/or a drug formulation, is deliverable to the substrate 102, and an outlet 114 at which fluid is permitted to leave the substrate 102, for example for the purpose of carrying waste away from the chamber unit(s) 110 and/or to enable replenishing of the cell nourishment and/or drug formulation.

A flow control system (not visible) can be used to supply the fluid to the inlet 112 and remove fluid from the fluidic device 100 via the outlet 114. Flow control systems for this purpose are commercially available, for example from Fluigent^{®}.

As shown in FIGs. 1A and 1B, an inlet fluid channel 116 is defined in the substrate 102 and extends between the inlet 112 and the at least one chamber unit 110, and an outlet fluid channel 118 is defined in the substrate 102 and extends between the at least one chamber unit 110 and the outlet 114. Thus, the inlet fluid channel 116 and the outlet fluid channel 118 fluidly connect the inlet 112 and the outlet 114 to the chamber unit(s) 110.

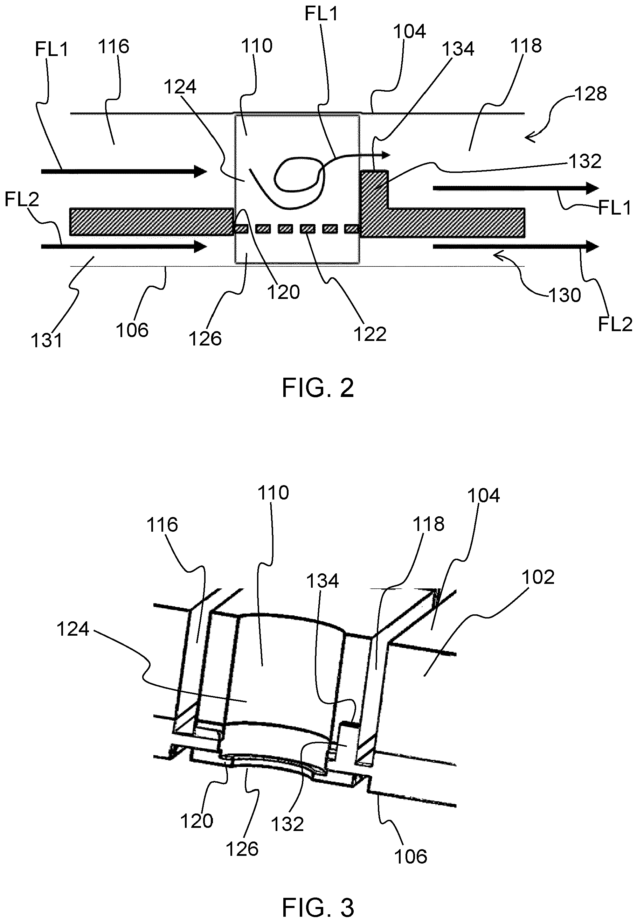

The biological material can be supported in the chamber unit(s) 110 in any suitable manner. In some embodiments, and referring now to FIG. 2, each of the at least one chamber unit 110 comprises at least one supporting region 120 for supporting a biocompatible partition 122 so that the biocompatible partition 122 separates an upper portion 124 from a lower portion 126 of the respective chamber unit 110. The biological material may be supported by the biocompatible partition 122 when the biological material is received in the chamber unit(s) 110.

In some embodiments, such as shown in FIG. 3, the supporting region(s) 120 comprise(s) a support ledge, provided around an inner perimeter of the respective chamber unit 110, on which the biocompatible partition 122 can be supported.

In at least some embodiments, the biocompatible partition 122 is suitable for culturing cells thereon. Such a biocompatible partition 122 is preferably in the form of a perforated membrane or film. In more general terms, the biocompatible partition 122 is preferably a biocompatible porous partition 122.

The pores of such a biocompatible porous partition 122 may assist the biocompatible partition 122 to have appropriate properties, in particular for cell culturing.

The biocompatible partition 122 can be formed from any suitable biocompatible material. Particular mention is made of the biocompatible partition 122 comprising an elastomeric material, for instance silicone rubber and/or polybutadiene.

Elastomeric materials for the biocompatible partition 122 are described, for example, in WO2021058657, WO2019015988.

Silicone and polybutadiene are readily functionalized, e.g. via cross-linking with fatty acids, to render them biocompatible, e.g. via application of a protein such as fibronectin to the fatty acid-functionalized surface of the elastomeric material. This, together with their elastic properties, makes such elastomeric materials particularly appropriate for inclusion in the biocompatible partition 122.

Silicone may be optically transparent and also have limited autofluorescence so that such the silicone-based biocompatible partition 122, e.g. membrane, may allow a variety of conventionally used optical inspection techniques and protocols, e.g. with or without staining, to be used in testing performed using the fluidic device 100.

The biocompatible partition 122 can be rendered porous, fluid-permeable, e.g. gas permeable, and/or suitable for cell culturing thereon in any suitable manner, for example by forming apertures in the material, e.g. elastomeric material, constituting the biocompatible partition 122. Such apertures can be formed in any suitable manner, for example by laser ablation or casting imprint perforation.

Laser perforation/ablation is described, for example, in US2014127744 and US20180315409. Casting imprint perforation is described, for instance, in US2020360923, US2015010919 and US2022228108.

Each of the apertures is preferably sized to restrict or prevent cells supported on the biocompatible partition 122 from passing therethrough. To this end, each aperture may have a diameter less than 10 µm.

In some embodiments, the material constituting the biocompatible partition 122 may be manufactured, e.g. by injection molding, as a slab or plate of material that is subsequently subjected to an aperture-forming process, e.g. comprising laser ablation. In this manner, several biocompatible partitions 122 may, for instance, be manufactured in parallel. The biocompatible partitions 122 may then be separated from each other, for example by being punched out of the slab, and inserted into the respective chamber unit 110 to be supported by the supporting region(s) 120.

In more general terms, the biocompatible partition 122 may be attachable to the substrate 102. For example, the biocompatible partition 122, e.g. membrane/film, may in the form of an insert that can be inserted into each chamber unit 110. This may mean that the biocompatible partition 122 and the substrate 102 are separately manufacturable, for example from different materials.

In embodiments in which the substrate 102 is formed from the same material(s) as the biocompatible partition 122, the fabrication process may comprise forming the substrate 102 and integral biocompatible partition 122, for example in a molding, e.g. injection molding, process, and subsequently forming the apertures in the integral biocompatible partition 122, e.g. via laser ablation.

The substrate 102 can be conveniently made from the same material as the biocompatible partition 122, e.g. membrane or film, in for example one injection molding step, especially when the biocompatible partition 122 has a thickness of at least 10 µm.

It is noted that injection molding tends to require the biocompatible partition's 122 thickness to be greater than 10 µm. Laser ablation may become more difficult to implement with increasing thickness of the biocompatible partition 122.

However, for thicknesses of 50 µm or less perforations in the biocompatible partition 122 can be conveniently made using laser ablation/perforation (see, for example, US4923608 and US20180315409).

A list of exemplary materials for the biocompatible partition 122 and/or the substrate 102 is provided herein below.

In some embodiments, the substrate 102 is formed from elastomeric material(s), e.g. silicone and/or polybutadiene.

In such embodiments, connecting the above-mentioned flow control system to the substrate 102, for example to supply fluid to the fluidic device 100 via the inlet 112 and remove fluid from the fluidic device 100 via the outlet 114, may be facilitated. This is because tubing for connecting the flow control system to the substrate 102 can be simply pressed into opening(s) defined in the elastomeric substrate 102, e.g. via a leak tight push-fit.

It is noted, for the avoidance of doubt, that such opening(s) defined in the substrate 102 may correspond to the inlet 112 and/or the outlet 114.

This pressing, e.g. push-fitting, may advantageously obviate the requirement for clamps and a cumbersome connection protocol in order to connect the tubing to the fluidic device 100.

It is noted that waste tubing for carrying waste away from the fluidic device 100 may typically have a relatively large diameter, e.g. larger than supply tubing for carrying cell nourishment and/or a drug formulation to the fluidic device 100. Accordingly, such waste tubing can be readily pressed into opening(s) that provide(s) fluid exit(s), e.g. the outlet 114.

It is noted that in embodiments in which the fluidic device 100 includes the upper cover and/or the lower cover, such cover(s) may delimit one or more holes through which the tubing can pass in order to connect the flow control system to the substrate 102.

In a closed system in which the flow control system is connected to the substrate 102, the connection of the tubing with the inlet 112 and the outlet 114 can be made, for example, with ferrules. Such ferrules can be made of elastomeric, e.g. soft elastomeric, materials.

Similar connections may be made in the case of so-called open systems but then the upper cover, e.g. upper cover plate, may be adapted to permit connection of the tubing with the inlet 112 and the outlet 114. In particular, the upper cover may delimit openings for the connections with the inlet 112 and the outlet 114.

Such an adapted upper cover may assist to avoid excessive evaporation of fluid, e.g. medium, from the chamber unit(s) 110, as well as assisting to minimize the risk of cross-contamination via aerosols in the manner of a standard cover for a well cell plate.

In embodiments in which the substrate 102 is formed from silicone, e.g. via injection molding, a glass upper cover and/or a glass lower cover may be adhered to the silicone substrate 102.

Adhesion between such a silicone substrate 102 and the glass upper cover and/or glass lower cover may be relatively strong. This may be particularly beneficial in embodiments in which a closed system in which one or both of the upper cover and lower cover is or are permanently secured to the substrate 102 is desired.

It is emphasized that the substrate 102 can be fabricated using any suitable technique. The substrate 102, e.g. together with the biocompatible partition 122, may be formed for example by injection molding or by an additive manufacturing process, such as 3D printing.

In the case of 3D printing, a thermoplastic elastomer may be used to manufacture the substrate 102 without the biocompatible partition 122, e.g. membrane.

In such embodiments, the thermoplastic elastomer may, for instance, be printed on an adhesive tape, such as Avery Dennison MED 1125.

The adhesive tape may, for example, be initially adhered to a glass substrate, e.g. plate, and the thermoplastic elastomer may be joined to the tape during the 3D printing.

Porous channel(s) constituting lower fluid channel(s) 131 of the lower fluid flow system 130 can be made, for example with a printed architecture as described in WO2023280808.

The biocompatible partition 122, e.g. membrane fabricated by injection molding and laser ablation to provide pores, may be inserted into the chamber unit(s) 110 of the printed substrate 102.

More generally, and referring again to FIG. 2, the fluidic device 100, and more particularly each of the one or more fluidic assemblies 108, may comprise an upper fluid flow system 128 defined in the substrate 102 and arranged to supply fluid and permit fluid to leave the upper portion 124 of the chamber unit(s) 110. Each of the one or more fluidic assemblies 108 may also comprise a lower fluid flow system 130 defined in the substrate 102 and arranged to supply fluid to and/or permit fluid to leave the lower portion 126 of the chamber unit(s) 110.

To this end, the lower fluid flow system 130 may include at least one lower fluid channel 131 arranged to supply fluid to and/or permit fluid to leave the lower portion 126 of the chamber unit(s) 110.

Referring to FIGs. 1B, 2 and 3, the at least one lower fluid channel 131 may, for instance, extend between a lower fluid inlet 131A and a lower fluid outlet 131B via the lower portion 126 of the chamber unit(s) 110. The flow control system may be connectable via tubing to the lower fluid inlet 131A and the lower fluid outlet 131B.

It is noted that the upper fluid system 128 may include the inlet fluid channel 116 and the outlet fluid channel 118. As shown in FIGs. 2 and 3, the upper portion 124 of the chamber unit(s) 110 may be arranged to receive fluid from the inlet fluid channel 116.

The biocompatible partition 122 may provide a separation between the top flow provided by the upper fluid flow system 128 and the bottom flow provided by the lower fluid flow system 130 that is non-permeable to the bottom flow.

The upper and lower fluid flow systems 128, 130 may fulfil different functions relative to each other, for example by the upper fluid flow system 128 supplying cell nourishment to the upper portion 124 of the chamber unit(s) 110, while the lower fluid flow system 130 supplies a drug formulation to the lower portion 126 of the chamber unit(s) 110.

To this end, the upper and lower fluid flow systems 128, 130 may be mutually independently controlled in respect of flow and/or composition of fluid to create various, e.g. the same or different, conditions in the upper portion 124 and the lower portion 126 of the chamber unit(s) 110.

In some embodiments, the upper fluid flow system 128 is employed to provide fluid flow(s) FL1 for transportation of cells, for instance mimicking blood flow in tissue of a subject, and/or fluid flow(s) comprising nourishment for cells being cultured. Alternatively or additionally, the lower fluid flow system 130 may be employed to provide fluid flow(s) FL2 for carrying drug or other treatments to the biological material, e.g. cell culture and/or metastasized cells.

In some embodiments, the upper and lower portions 124, 126 have different volumes relative to each other, for example with the lower portion 126 having a smaller volume than that of the upper portion 124. An example of this is shown in FIGs. 2 and 3. This may be beneficial when testing a drug formulation that is more appropriately supplied, e.g. for cost and/or scarcity of supply reasons, to whichever of the upper and lower portions 124, 126 has the smaller volume.

Alternatively, or additionally, the at least one supporting region 120 may be arranged to support the biocompatible partition 122 closer to one of the top and bottom sides 104, 106 than the other of the top and bottom sides 106, 104. In some embodiments, such as shown in FIGs. 2 and 3, the supporting region(s) 120 is or are configured to support the biocompatible partition 122 closer to the bottom side 106 than to the top side 104. This may facilitate monitoring of the biological material, particularly by optical microscopy, e.g. through the above-mentioned optically transmissive lower or upper cover.

It is noted that the focus distance of many optical microscopy set-ups, e.g. employed for imaging stainings, may only be about 0.6 mm. The distance from the bottom of the lower cover, e.g. glass lower cover, and the biocompatible partition 122, e.g. perforated membrane, may accordingly be not more than 0.6 mm.

Thus, in the case of, for example, a lower cover, e.g. glass lower cover, having a thickness of 0.20 mm, a distance between the lower cover and the biocompatible partition 122 may be at most 0.4 mm.

Accordingly in some embodiments, the supporting region(s) 120 is or are configured to support the biocompatible partition 122 at a distance not larger than 0.4 mm from the bottom side 106 of the substrate 102.

More generally, and still referring to FIGs. 2 and 3, fluid flow through the at least one chamber unit 110 is controllable by a dam assembly, with the dam assembly shown in these particular Figures including an outlet dam 132 between the at least one chamber unit 110 and the outlet fluid channel 118 and upstanding towards the top side 104 to an outlet threshold 134. The outlet threshold 134 is arranged to permit fluid from the at least one chamber unit 110 to flow thereover to be received in the outlet fluid channel 118.

The outlet dam 132 can assist to collect fluid, e.g. cell culture medium, over the biological material, e.g. cell culture, before the fluid leaves the substrate 102 via the outlet 114.

In some embodiments, the outlet dam 132 can create larger fluid reservoir(s), e.g. for providing cell nourishment. Particularly since the fluid, e.g. flow medium, may be constantly refreshed, for instance via operation of the flow control system, sufficient oxygen/carbon dioxide assimilation may be ensured. By shaping, e.g. selecting the height of, the outlet threshold 134, larger fluid reservoir(s) can be provided.

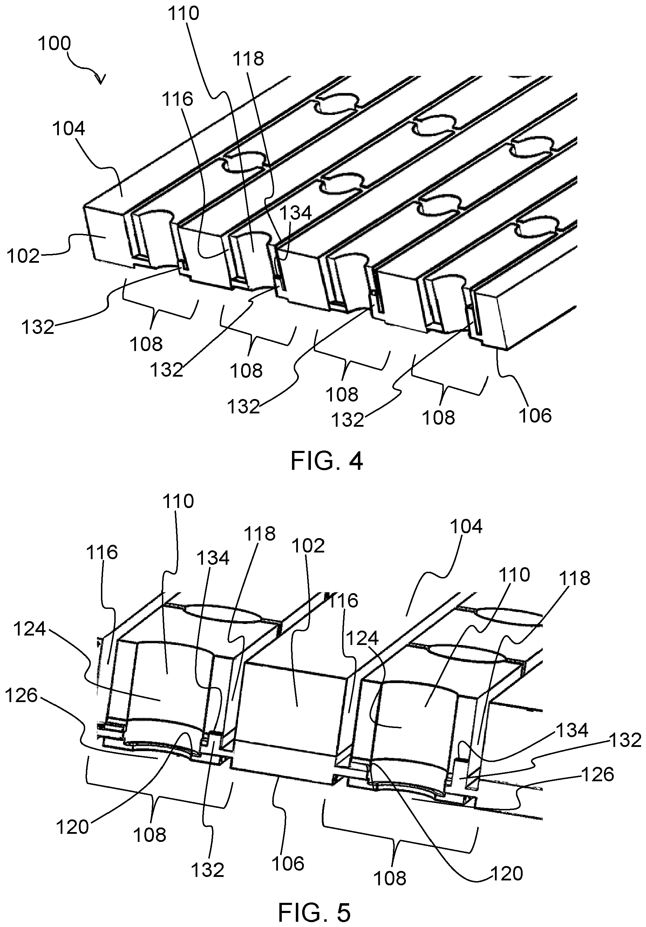

In embodiments in which the fluidic device 100 comprises a plurality of the fluidic assemblies 108, such as shown in FIGs. 4 and 5, the outlet thresholds 134 of at least some of the plurality of fluidic assemblies 108 may be at different heights relative to each other.

Such a fluidic device 100 may enable testing to identify a suitable height of the outlet threshold 134, e.g. as part of process to optimize cell culturing conditions. For example, a 3D printed fluidic device 100 in which outlet thresholds 134 of at least some of the plurality of fluidic assemblies 108 are at different heights relative to each other may be used for such testing. Injection molded fluidic device(s) 100 may be subsequently produced, whose outlet threshold(s) 134 is or are set based on the results of the testing using the 3D printed fluidic device 100.

In the non-limiting example shown in FIG. 4, the height of the outlet threshold 134 of the outlet dam 132 of the far left hand fluidic assembly 108 is 1 mm, the height of the outlet threshold 134 of the outlet dam 132 of the middle left hand fluidic assembly 108 is 2 mm, the height of the outlet threshold 134 of the outlet dam 132 of the middle right hand fluidic assembly 108 is 3 mm, and the height of the outlet threshold 134 of the outlet dam 132 of the far right hand fluidic assembly 108 is 4 mm.

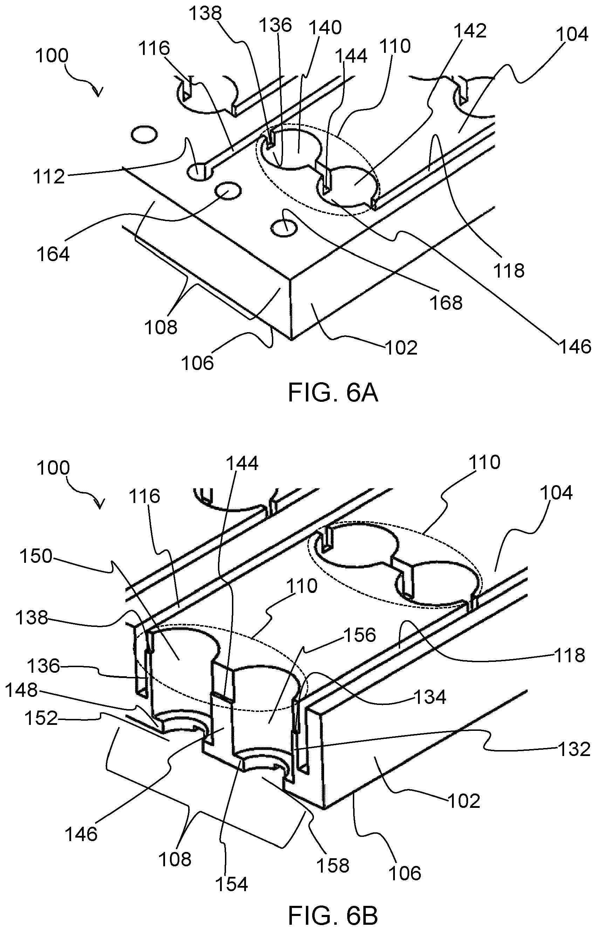

In some embodiments, such as shown in FIGs. 6A to 6C, the dam assembly comprises an inlet dam 136 between the inlet fluid channel 116 and the at least one chamber unit 110 and upstanding towards the top side 104 to an inlet threshold 138. The inlet threshold 138 is arranged to permit fluid from the inlet fluid channel 116 to flow thereover to be received in the at least one chamber unit 110.

The inlet fluid channel 116 may be configured to fill up to the inlet threshold 138 prior to the fluid flowing over the inlet threshold 138 to enter the chamber unit(s) 110.

Such inlet dams 136 can in some embodiments enable stepwise filling of chamber units 110, e.g. one by one. This may obviate tilting of the substrate 102, e.g. by 5 to 10 degrees from the horizontal, in order to implement such stepwise filling of the chamber units 110.

In some embodiments, such as shown in FIGs. 6A to 6C, the at least one chamber unit 110 extends to an opening at the top side 104 of the substrate 102, and the dam assembly comprises the inlet dam 136 and the outlet dam 132, with the inlet dam 136 and the outlet dam 132 being arranged to retain fluid in the at least one chamber unit 110.

In such embodiments, the fluidic device 100 may be operable as a so-called open system in which fluid delivery into and/or fluid withdrawal from the at least one chamber unit 110 can be implemented, e.g. via a pipette arrangement, without having to connect a flow control system to the inlet 112 and the outlet 114. This capability is due to the chamber unit(s) 110 retaining fluid therein irrespective of whether or not there is flow of fluid being provided via the inlet fluid channel 116.

In some embodiments, and still referring to FIGs. 6A to 6C, the outlet threshold 134 of the outlet dam 132 is lower than the inlet threshold 138 of the inlet dam 136 so that fluid filling the at least one chamber unit 110 to reach the outlet threshold 134 flows over the outlet threshold 134 and into the outlet fluid channel 118.

This arrangement enables the fluidic device 100 to be additionally utilizable as a closed system in which the flow control system drives flow in the inlet fluid channel 116 and the outlet fluid channel 118. By the outlet threshold 134 being lower than the inlet threshold 138, the risk of undesirable backflow may be minimized.

In other embodiments, the outlet threshold 134 is higher than the inlet threshold 138.

This may assist to provide sufficient fluid, e.g. nourishment, to biological material in the chamber unit(s) 110.

In some embodiments, such as shown in FIGs. 1A, 1B and 6A to 6C, a dual chamber unit 110 is included in one or more of the at least one chamber unit 110, with the dual chamber unit 110 comprising a first chamber 140 and a second chamber 142 fluidly connected to each other via a bridge 144 defined between the first chamber 140 and the second chamber 142. Such a dual chamber unit 110 may have utility in studying metastasis of cancerous cells. It may be possible to assess cells that are released or emitted by any cell cultures that are provided in the first chamber 140. The first chamber 140 can therefore be used to perform a treatment on the cell culture(s), such as the application of a certain therapeutic fluid or drug, for example a chemotherapy treatment fluid, with the second chamber 142 capturing any released cells to analyze the effect of the treatment performed in the first chamber 140 on metastasis.

In such embodiments, the inlet dam 136 may be between the inlet fluid channel 116 and the first chamber 140 so that fluid filling the inlet fluid channel 116 to reach the inlet threshold 138 flows over the inlet threshold 138 into the first chamber 140.

Moreover, the outlet dam 132 may be between the second chamber 142 and the outlet fluid channel 118.

The fluidic device 100, and more particularly each of the one or more fluidic assemblies 108, may further comprise an intermediate dam 146 between the first and second chambers 140, 142, with the intermediate dam 146 upstanding towards the top side 104 of the substrate 102 to a bridge threshold at a base of the bridge 144 between the first chamber 140 and the second chamber 142.

In such embodiments, the bridge threshold is preferably lower than the inlet threshold 138 so that fluid filling the first chamber 140 to reach the bridge threshold is permitted to flow over the bridge threshold into the second chamber 142. Moreover, the outlet threshold 134 is preferably lower than the bridge threshold so that fluid filling the second chamber 142 to reach the outlet threshold 134 flows over the outlet threshold 134 into the outlet fluid channel 118.

This ordering of heights of the inlet threshold 138, the bridge threshold and the outlet threshold 134 may assist to minimize the risk of undesirable backflow during closed system-type operation of the fluidic device 100 using the fluid control system. It is also reiterated that removing fluid, e.g. old fluid, from and adding fresh fluid to the chamber unit(s) 110 can also be achieved via pipetting.

In the non-limiting example shown in FIGs. 6A to 6C, the height of the inlet threshold 138 is 5 mm, the height of the bridge threshold is 4 mm, and the height of the outlet threshold 134 is 3 mm.

In other embodiments, the bridge threshold is higher than the inlet threshold 138.

This may assist to provide sufficient fluid, e.g. nourishment, to biological material in the first chamber 140.

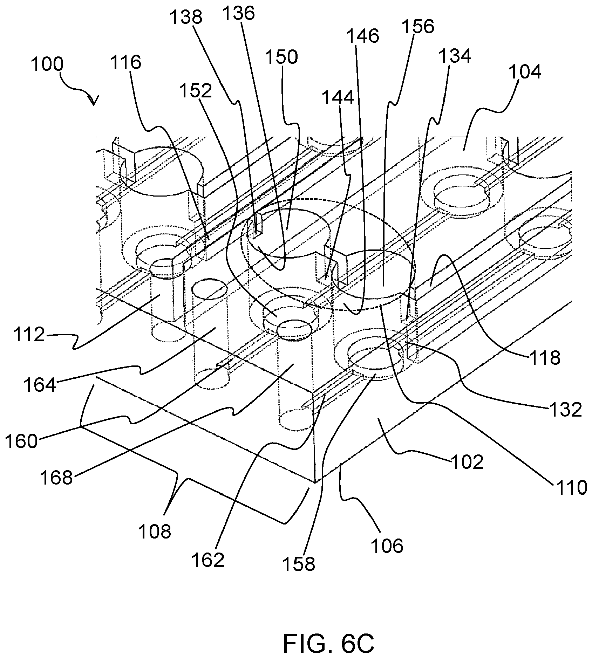

In some embodiments, and referring to FIGs. 6B and 6C, the at least one supporting region 120 comprises a first supporting region 148 for supporting a first biocompatible partition (not visible) so that the first biocompatible partition separates a first upper portion 150 from a first lower portion 152 of the first chamber 140.

In such embodiments, the at least one supporting region 120 may further comprise a second supporting region 154 for supporting a second biocompatible partition (not visible) so that the second biocompatible partition separates a second upper portion 156 from a second lower portion 158 of the second chamber 142.

In embodiments in which the one or more fluidic assemblies 108 each comprise a plurality of chamber units 110 that each comprise the first chamber 140 and the second chamber 142, and referring now to FIG. 6C, the lower fluid flow system 130 may comprise a first lower fluid channel 160 arranged to fluidly connect the first lower portions 152 of the first chambers 140 to each other, and a second lower fluid channel 162 arranged to fluidly connect the second lower portions 158 of the second chambers 142 to each other. Such an arrangement may assist to prevent blockages in the fluidic device 100.

It is noted more generally that the underflow provided by the lower fluid flow system 130 may be stopped or refreshed continuously depending on the protocol designed/desired by the experimentalist, e.g. oncologist. Since the biocompatible partition 120 may be impermeable for cells, e.g. due to the limited size of apertures formed therein, as previously described, there may be little or no risk of contamination of other chamber unit(s) 110 via the underflow. If chemicals for chemotherapy are used for the underflow, cross-contamination of proteins may also be minimized because of the relatively small overpressure on the lower fluid flow system 130.

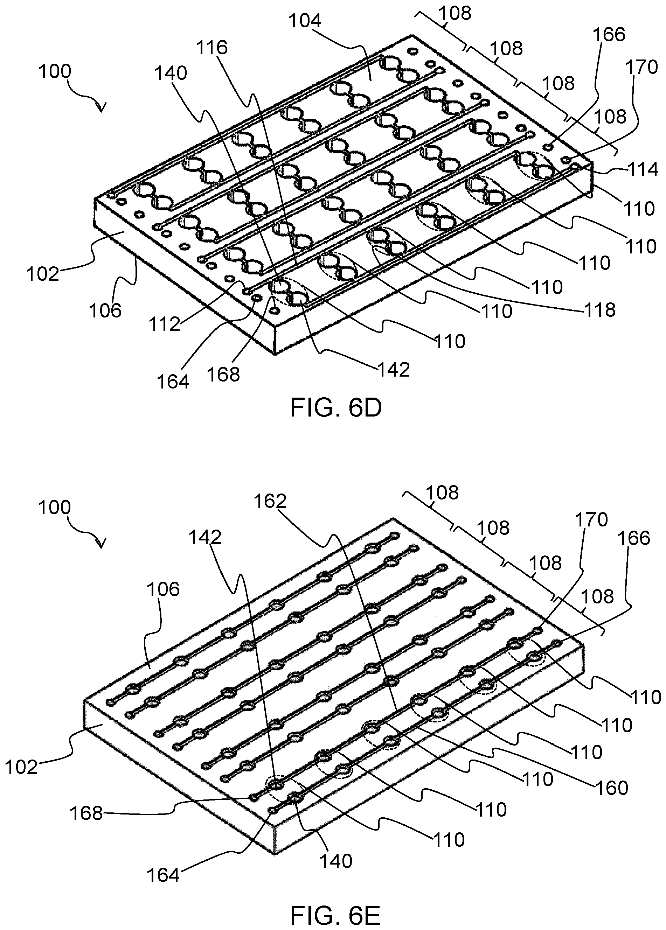

Referring to FIGs. 6D and 6E, the first lower fluid channel 160 may, for instance, extend between a first lower fluid inlet 164 and a first lower fluid outlet 166. The flow control system may be connectable via tubing to the first lower fluid inlet 164 and the first lower fluid outlet 166.

Similarly, the second lower fluid channel 162 may extend between a second lower fluid inlet 168 and a second lower fluid outlet 170.

At this point it is noted that the chamber unit(s) 110, for example the chamber or chambers 140, 142 constituting each of the chamber unit(s) 110, may have dimensions suitable for the intended testing of the biological material. In some embodiments, each chamber unit 110, for example each chamber 140, 142 thereof, may have a height extending between the top side 104 and the bottom side 106 of 1.8 to 2.6 mm, e.g. about 2.2 mm. Alternatively or additionally, a diameter of each chamber unit 110, for example each chamber 140, 142 thereof, may be 3 to 20 mm, such as 3 to 10 mm, e.g. about 6 mm.

Such dimensions may balance the requirement for the chamber unit(s) 110 to be kept as small as possible, e.g. to enable as many chamber unit(s) 110 to be defined in the substrate 102 as possible, with the requirement for the chamber unit(s) 110 to provide sufficient space for testing of the biological material, e.g. for capture and subsequent analysis of cells.

The chamber unit(s) 110, for example the chamber or chambers 140, 142 constituting each of the chamber unit(s) 110, may have any suitable shape. A generally cylindrical shape may be preferred from a fluid dynamics perspective, e.g. to mitigate the risk of pooling of fluid in a corner of a chamber unit 110, e.g. in a corner of the chamber(s) 140, 142 thereof, and/or to ensure usability with existing analysis equipment. However, shapes other than cylindrical can be contemplated, e.g. shapes having a square or rectangular cross-section.

It is also noted that a depth of the inlet fluid channel 116 and/or the outlet fluid channel 118 may be 0.5 to 1.5 mm, e.g. about 0.8 mm. A width of the inlet fluid channel 116 and/or the outlet fluid channel 118 may be 0.5 to 1.5 mm, e.g. about 1 mm.

A depth of each of the lower fluid channel(s) 131 may be 0.2 to 0.8 mm, e.g. about 0.6 mm; and/or a width of each of the lower fluid channel(s) 131 may be 0.5 to 1.5 mm, e.g. about 1 mm.

It is also reiterated that the distance from the bottom of the lower cover, e.g. glass lower cover, and the biocompatible partition 122, e.g. perforated membrane, may be not more than 0.6 mm.

With reference to FIGs. 6A to 6E, as well as FIGs. 7A, 7B, 8, 9A and 9B, in embodiments in which each of the one or more fluidic assemblies 108 includes a plurality of chamber units 110, the chamber units 110 may be connected in parallel across the inlet fluid channel 116 and the outlet fluid channel 118. Such a design can facilitate efficient connection to the flow control system due to fewer connection tubing points being required, whilst also assisting to minimize cross-contamination between chamber units 110.

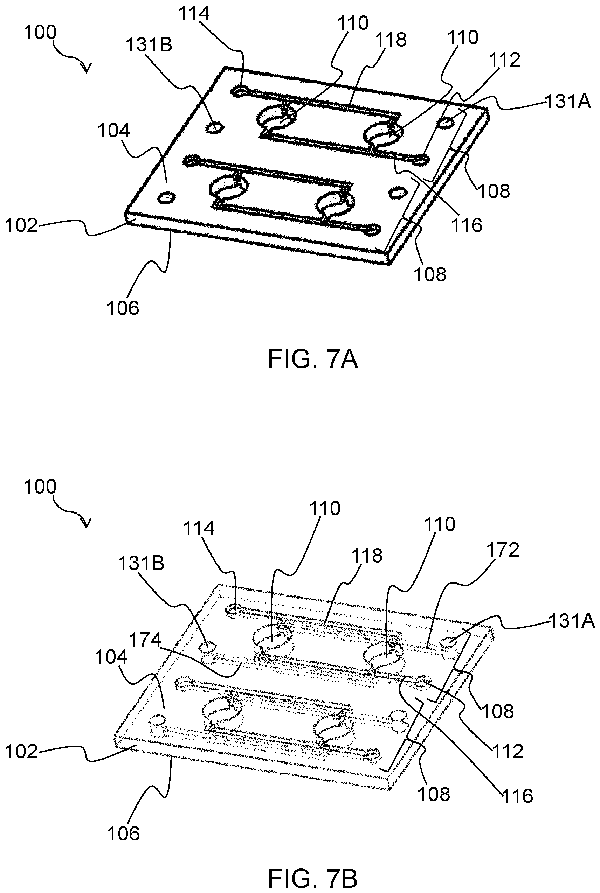

Alternatively or additionally, and referring to FIG. 7B, the lower flow control system 130 may, for example, include a lower inlet fluid channel 172 and a lower outlet fluid channel 174, with the lower portions 126; 152, 158 of the chamber units 110 being connected in parallel across the lower inlet fluid channel 172 and the lower outlet fluid channel 174.

This arrangement may be effective in reducing the risk of blockages in the fluidic device 100.

Whilst not visible in the Figures, the inlet fluid channel 116 may provide a resistance to flow therein that changes with distance along the inlet fluid channel 116 away from the inlet 112, for example by variation of the dimensions, e.g. cross-sectional area, of the inlet fluid channel 116 with distance along the inlet fluid channel 116 away from the inlet 112.

In such embodiments, a narrower portion of the inlet fluid channel 116 may be provided proximal to the inlet 112, followed by a wider portion of the inlet fluid channel 116 along the inlet fluid channel 116 further away from the inlet 112.

In embodiments in which the chamber units 110 are connected in parallel across the inlet fluid channel 116 and the outlet fluid channel 118, the resistance to flow in the inlet fluid channel 116 may decrease, for example due to the inlet fluid channel 116 having increased cross-sectional area, between the fluid inlets, e.g. inlet thresholds 138, of successive chamber units 110 in a downstream direction away from the inlet 112.

Such a design of the inlet fluid channel 116 may assist to control, e.g. provide equal continuous flow, across each of the parallel chamber units 110.

Alternatively or additionally, the outlet fluid channel 118 may provide a resistance to flow therein that changes with distance along the outlet fluid channel 118 away from the fluid outlets, e.g. outlet thresholds 134, of the chamber unit(s) 110, for example by variation of the dimensions, e.g. cross-sectional area, of the outlet fluid channel 118 with distance along the outlet fluid channel 118 away from the fluid outlets.

It is noted that FIGs. 7A and 7B depict a fluidic device 100 comprising two parallel connected chamber units 110, with the chamber units 110 each being a single chamber, per fluidic assembly 108. Moreover in this case, the fluidic device 100 includes a pair of fluidic assemblies 108. This example may be simpler than that of the fluidic device 100 shown in FIGs. 6D and 6E, in which there are six parallel connected dual-chamber units 110 per fluidic assembly 108, and with the fluidic device 100 comprising four fluidic assemblies 108.

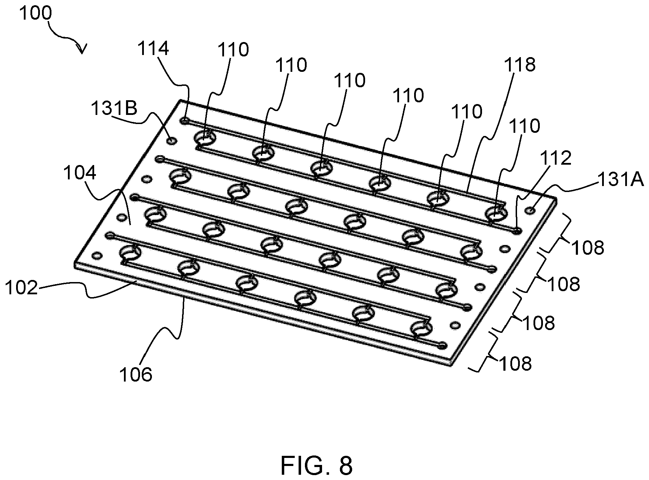

FIG. 8 depicts a fluidic device 100 comprising six parallel connected chamber units 110, with the chamber units 110 each being a single chamber, per fluidic assembly 108. In this case, the fluidic device 100 includes four fluidic assemblies 108.

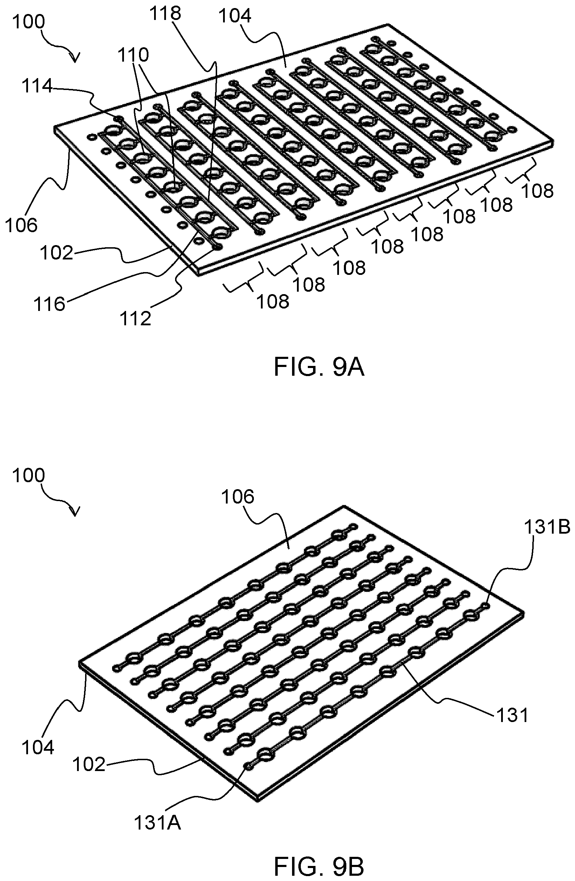

In embodiments in which the fluidic device 100 comprises a plurality of fluidic assemblies 108 that each include chamber units 110 connected in parallel across the inlet fluid channel 116 and the outlet fluid channel 118, the lower fluid flow system 130 may include lower fluid channels 131 that each provide a fluid connection between lower portions 126 of chamber units 110 belonging to different fluidic assemblies 108. A non-limiting example of this is shown in FIGs. 9A and 9B. This may reduce the risk of blockages in the fluidic device 100.

FIGs. 9A and 9B depict a fluidic device 100 comprising eight parallel connected chamber units 110, with the chamber units 110 each being a single chamber, per fluidic assembly 108. In this case, the fluidic device 100 includes eight fluidic assemblies 108.

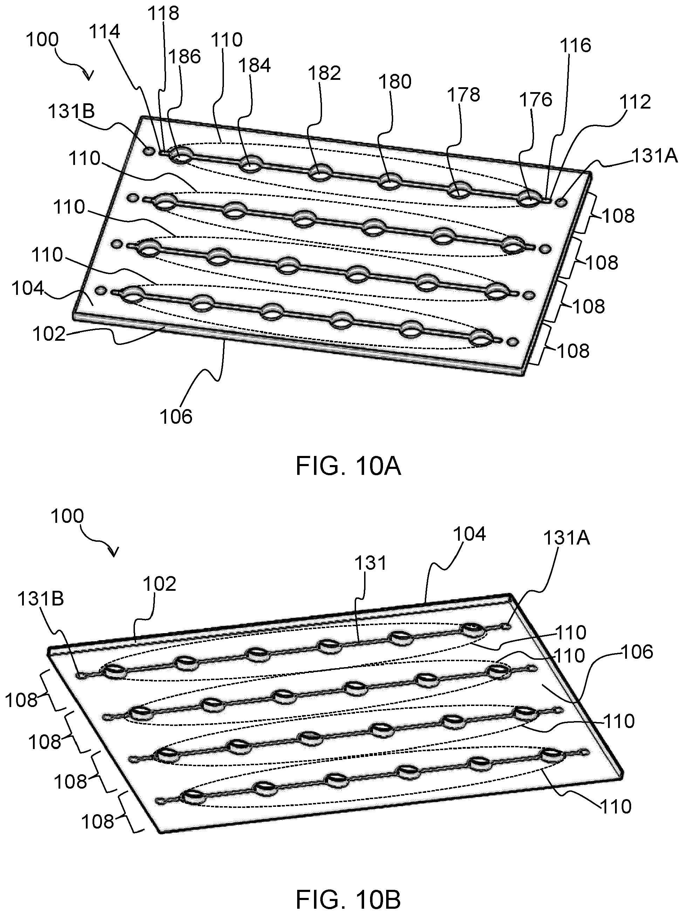

In some embodiments, such as shown in FIGs. 10A and 10B, each of the one or more fluidic assemblies 108 includes a chamber unit 110 comprising more than two, and in this example six, chambers 176, 178, 180, 182, 184, 186 connected in series.

Such embodiments can be regarded as an elaboration on the embodiments described above in which each chamber unit 110 comprises the first chamber 140 and the second chamber 142.

The fluidic device 100 shown in FIG. 8 has a twenty four chamber, e.g. well, configuration, the fluidic device 100 shown in FIGs. 9A and 9B has a sixty four chamber, e.g. well, configuration, and the fluidic device 100 shown in FIGs. 10A and 10B has a twenty four chamber, e.g. well, configuration. It is noted that the substrate 102 of the fluidic device 100 may have a shape and size that corresponds with the size and shape of well plates that are well-known and generally used in the field. Well plates having ninety six chambers are also known and can be contemplated in the context of the fluidic device 100 according to the present disclosure.

More generally, the present disclosure proposes use of the fluidic device 100 according to any of the embodiments described herein for testing biological material, for example for testing cell groups, double cell layers, spheroids, organoids or biopsies.

The disclosure further contemplates a drug testing method comprising providing the fluidic device 100 according to any of the embodiments described herein, receiving biological material in the at least one chamber unit 110, and exposing the biological material to the drug to be tested, e.g. by delivering the drug to be tested using one or both of the upper and lower fluid flow system(s) 128, 130.

In some embodiments, the receiving of biological material in the at least one chamber unit 110 comprises culturing cells in the at least one chamber unit 110.

Alternatively or additionally, the method may comprise monitoring a response of the biological material, e.g cultured cells, to the drug to be tested. This monitoring may be implemented in any suitable manner, for example by optical microscopy, e.g. through the above-mentioned optically transmissive lower or upper cover.

The following exemplary commercially available elastomeric/soft materials (which are transparent or translucent and are food contact approved) can be used, in certain embodiments, as the material for the biocompatible partition 122, the substrate 102 and/or other components of the fluidic device 100: Medalist MD-53253 (TPE Teknor Apex); Medalist MD-53273 (TPE Teknor Apex); Mediprene 500602 M-03 (TPE Hexpol); Texin Rx T85A (TPU Covestro); BioSpan^{®} (F SPU | PUR); BioSpan^{®} (SPU | PUR); BJB Polyurethane F-116 A/B | TSU; BJB Polyurethane F-126 A/B | TSU; BJB Polyurethane F-131 A/B | TSU; BJB Polyurethane M-3115 REV 1 A/B | TSU; BJB Polyurethane M-3125 A/B | TSU; CELLENE MC2248 | TPE; CELLENE MC2265 | TPE; CELLENE MC3038 | TPE; CELLENE MC3050 | TPE; CELLENE MC3061 | TPE; CELLENE MC3226 | TPE; CELLENE MC3239 | TPE; CELLENE MC3261 | TPE; Dynaflex^{™} G2706-1000-00 | TPE; Dynaflex^{™} G2711-1000-00 | TPE; Elastocon^{®} 2860L | TPE; Filter-bond^{™} E-3264 | TS; FLEXCHEM^{™} 3551-02 | PVC, Flexible; FLEXCHEM^{™} 4051-02 | PVC, Flexible; FLEXCHEM^{™} 4551-02 | PVC, Flexible; FLEXCHEM^{™} 5051-02 | PVC, Flexible; FLEXCHEM^{™} 5551-02 | PVC, Flexible; FLEXCHEM^{™} 6051-02 | PVC, Flexible; FLEXCHEM^{™} 6551-02 | PVC, Flexible; Medalist^{®} MD-12130 | TPE; Medalist^{®} MD-12130H | TPE; Medalist^{®} MD-12140 | TPE; Medalist^{®} MD-12140H | TPE; Medalist^{®} MD-12150 | TPE; Medalist^{®} MD-12150H | TPE; Medalist^{®} MD-12f150S | TPE; Medalist^{®} MD-12160 | TPE; Medalist^{®} MD-12160H | TPE; Medalist^{®} MD-12170 | TPE; Medalist^{®} MD-12170H | TPE; Medalist^{®} MD-12243 | TPE; Medalist^{®} MD-12337 | TPE; Medalist^{®} MD-12340 NAT | TPE; Medalist^{®} MD-12342 | TPE; Medalist^{®} MD-12344 | TPE; Medalist^{®} MD-12350 | TPE; Medalist^{®} MD-12352 | TPE; Medalist^{®} MD-12362 | TPE; Medalist^{®} MD-125 | TPE; Medalist^{®} MD-130 | TPE; Medalist^{®} MD-13240 | TPE; Medalist^{®} MD-135 | TPE; Medalist^{®} MD-145 | TPE; Medalist^{®} MD-155 | TPE; Medalist^{®} MD-17365 | TPE; Medalist^{®} MD-225 | TPV; Medalist^{®} MD-32045 | TPE; Medalist^{®} MD-32245 | TPE; Medalist^{®} MD-36048 | TPE; Medalist^{®} MD-37063 NAT | TPE; Medalist^{®} MD-42245 XRD1 | TPE; Medalist^{®} MD-42245 XRD3 | TPE; Medalist^{®} MD-74357 XRD1 | TPE; Medalist^{®} MD-74357 XRD2 | TPE; Mediprene^{®} 500120M | TPE; Mediprene^{®} 500200M | TPE; Mediprene^{®} 500250M | TPE; Mediprene^{®} 500300M | TPE; Mediprene^{®} 500350M | TPE; Mediprene^{®} 500400M | TPE; Mediprene^{®} 500434M | TPE; Mediprene^{®} 500450M | TPE; Mediprene^{®} 500484M | TPE; Mediprene^{®} 500520M | TPE; Mediprene^{®} 500534M | TPE; Mediprene^{®} 500584M | TPE; Mediprene^{®} 500600M | TPE; Mediprene^{®} 500634M | TPE; Mediprene^{®} 500650M | TPE; Mediprene^{®} 500684M | TPE; Mediprene^{®} 500700M | TPE; Monprene^{®} RG-10160H | TPE; ProvaMed^{®} TPE 1120 | TPE; ProvaMed^{®} TPE 1160 | TPE; RABALON^{®} PJ4300C | TPE; RABALON^{®} PJ5300C | TPE; RABALON^{®} PJ6300C | TPE; RABALON^{®} PJ7300C | TPE; SkinFlex 15 F-115 A/B | TSU; SkinFlex BR-60; BRUSHABLE A/B | TSU; T-Blend^{®} TPE-F22 | SEBS; THERMOLAST^{®} M TM3LFT (Series: MC/LF) | TPE; THERMOLAST^{®} M TM3MED (Series: MC/tl) | TPE; THERMOLAST^{®} M TM3RST (Series: MC/RS) | TPE; THERMOLAST^{®} M TM4LFT (Series: MC/LF) | TPE; THERMOLAST^{®} M TM4MED (Series: MC/tl) | TPE; THERMOLAST^{®} M TM4RST (Series: MC/RS) | TPE; THERMOLAST^{®} M TM5LFT (Series: MC/LF) | TPE; THERMOLAST^{®} M TM5MED (Series: MC/tl) | TPE; THERMOLAST^{®} M TM6LFT (Series: MC/LF) | TPE; THERMOLAST^{®} M TM6MED (Series: MC/tl) | TPE; THERMOLAST^{®} M TM7LFT (Series: MC/LF) | TPE
THERMOLAST^{®} M TM7MED (Series: MC/tl) | TPE; UNISOFT SPECIAL^{™} DS-35A-CL-M-01 | SEBS; UNISOFT SPECIAL^{™} DS-55A-CL-M-01 | SEBS; Versaflex^{™} G2705 N | TPE; Versaflex^{™} HC 1100-40 Translucent EU | TPE; Versaflex^{™} HC 1348 Natural | TPE; Versaflex^{™} HC MT317 | TPE; Versaflex^{™} HC MT555 | TPE; Versaflex^{™} OM 1040X-1 | TPE; CELLENE MC2248 | TPE; CELLENE MC2265 | TPE; CELLENE MC3038 | TPE; CELLENE MC3050 | TPE; CELLENE MC3061 | TPE; CELLENE MC3226 | TPE; CELLENE MC3239 | TPE; CELLENE MC3261 | TPE; ChronoPrene^{™} 25A | TPE; ChronoPrene^{™} 40A | TPE (CardioTech International, Inc.); Dryflex^{®} 500300S | TPE; Dryflex^{®} 500350S | TPE; Dryflex^{®} 500400S | TPE; Dryflex^{®} 500450S | TPE; Dryflex^{®} 500500S | TPE; Dryflex^{®} 500550S | TPE; Dryflex^{®} 500600S | TPE; Dryflex^{®} 500650S | TPE; Dryflex^{®} 500700S | TPE; Dynaflex^{™} G2701-1000-02 | TPE; Dynaflex^{™} G2706-1000-00 | TPE; Dynaflex^{™} G2709-1000-00 | TPE; Dynaflex^{™} G2711-1000-00 | TPE; Dynaflex^{™} G2712-1000-02 | TPE; Dynaflex^{™} G2730 | TPE; Dynaflex^{™} G2755-1000-00 | TPE; Dynaflex^{™} G2755C | TPE; Dynaflex^{™} G6713-0001 | TPE; Dynaflex^{™} G6713C | TPE; Dynalloy^{™} GP 7810-60T | TPE; Dynalloy^{™} GP 7810-70T | TPE; Dynalloy^{™} OBC8200-BT50 | TPE; Estane^{®} 58123 TPU | TPU-Polyether; Evoprene^{™} 019 | SBS; Evoprene^{™} G 925 | SEBS; Evoprene^{™} G 936 | SEBS; Evoprene^{™} G 942 | SEBS; Evoprene^{™} G 958 | SEBS; Evoprene^{™} G 966 | SEBS; Evoprene^{™} G 967 | SEBS; Evoprene^{™} G 968 | SEBS; Evoprene^{™} G 969 | SEBS; Evoprene^{™} G 970 | SEBS; Evoprene^{™} GC 5685 | SEBS; Evoprene^{™} GC 5686 | SEBS; Evoprene^{™} GC 5687 | SEBS; Evoprene^{™} GC 5688 | SEBS; Evoprene^{™} GC 5689 | SEBS; Evoprene^{™} GC 5690 | SEBS; GLS 422-126 | TPE; GLS 458-140 | TPE; GLS 458-141 | TPE; GLS 458-142 | TPE; K-Prene HYFLEX HF 15 | MPR; K-Prene HYFLEX HF 20 | MPR; K-Prene HYFLEX HF 25 | MPR; K-Prene HYFLEX HF 30 | MPR; Medalist^{®} RG-38052 XRD1 | TPE; megol^{®} PUG 10 | SEBS; megol^{®} PUG 60 | SEBS; megol^{®} TA 60 | SEBS; Monprene^{®} RG-10130 | TPE; Monprene^{®} RG-10140 | TPE; Monprene^{®} RG-10150 | TPE; Monprene^{®} RG-10160 | TPE; Monprene^{®} RG-10170 | TPE; Monprene^{®} RG-15130 | TPE; Monprene^{®} RG-15140 | TPE; Monprene^{®} RG-15150 | TPE; Monprene^{®} RG-15160 | TPE; Monprene^{®} RG-15170 | TPE; Monprene^{®} RG-18240 | TPE; Monprene^{®} RG-18250 | TPE; Monprene^{®} RG-18260 | TPE; Monprene^{®} RG-18270 | TPE; Monprene^{®} RG-19221 NAT | TPE; Monprene^{®} RG-19255 | TPE; Monprene^{®} RG-20140 | TPE; Monprene^{®} RG-20160 | TPE; Monprene^{®} RG-20170 | TPE; Monprene^{®} RG-29068 NAT | TPE; Monprene^{®} RG-29240 XRD1 | TPE; RABALON^{®} MJ4300C | TPE; RABALON^{®} MJ5302C | TPE; RABALON^{®} MJ6301C | TPE; RABALON^{®} MJ7301C | TPE; RAYPRENE^{®} NB221-S4050 | TPE; RAYPRENE^{®} NB221-S4051 | TPE; RAYPRENE^{®} NB221-S4052 | TPE; RAYPRENE^{®} NB221-S4053 | TPE; tefabloc^{®} TO 132 | TPE; Telcar^{®} TL-83-F943D22-NT BLU | TPE; THERMOLAST^{®} K TF2CGT (Series: FC) | TPE; THERMOLAST^{®} K TF3BTL (Series: FC/AP) | TPE; THERMOLAST^{®} K TF3CGT (Series: FC) | TPE; THERMOLAST^{®} K TF3STE (Series: FC/CS) | TPE; THERMOLAST^{®} K TF4AAB (Series: FC/HE/tl) | TPE; THERMOLAST^{®} K TF4BTL (Series: FC/AP) | TPE; THERMOLAST^{®} K TF4CGT (Series: FC) | TPE; THERMOLAST^{®} K TF4STE (Series: FC/CS) | TPE; THERMOLAST^{®} K TF5AAC (Series: FC/HE/tl) | TPE; THERMOLAST^{®} K TF5BTL (Series: FC/AP) | TPE; THERMOLAST^{®} K TF5CGT (Series: FC) | TPE; THERMOLAST^{®} K TF5STE (Series: FC/CS) | TPE; THERMOLAST^{®} K TF5WHA (Series: DW/H) | TPE; THERMOLAST^{®} K TF6AAF (Series: FC/HE/tl) | TPE; THERMOLAST^{®} K TF6BTL (Series: FC/AP) | TPE; THERMOLAST^{®} K TF6CGT (Series: FC) | TPE; THERMOLAST^{®} K TF6STE (Series: FC/CS) | TPE; THERMOLAST^{®} K TF6WCS (Series: DW/CS) | TPE; THERMOLAST^{®} K TF6WHA (Series: DW/H) | TPE; THERMOLAST^{®} K TF6WHB (Series: DW/H) | TPE; THERMOLAST^{®} K TF7AAC (Series: FC/HE/tl) | TPE; THERMOLAST^{®} K TF7BTL (Series: FC/AP) | TPE; THERMOLAST^{®} K TF7CGT (Series: FC) | TPE; THERMOLAST^{®} K TF7WHB (Series: DW/H) | TPE; Topolymer^{®} 8201-B | TPE; Versaflex^{™} FFC 2882-50 EU | TPE; Versaflex^{™} FFC 2882-50 | TPE; Versaflex^{™} G2708 N | TPE; Versaflex^{™} GP 2810-20N | TPE; Versaflex^{™} GP 2810-30N | TPE; Versaflex^{™} GP 2810-40N | TPE; Versaflex^{™} GP 2810-50N | TPE; Versaflex^{™} GP 2810-60N | TPE; Versaflex^{™} GP 2810-70N | TPE; Cawiton^{®} MT920 | SEBS; Cawiton^{®} MT930 | SEBS; Cawiton^{®} MT940 | SEBS; Cawiton^{®} MT950 | SEBS; Cawiton^{®} MT960 | SEBS; Cawiton^{®} MT970 | SEBS.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to".

Any reference signs in the claims should not be construed as limiting the scope.

## Claims

1. A fluidic device (100) for testing biological material, the fluidic device comprising:
a substrate (102) having a top side (104) and a bottom side (106); and
one or more fluidic assemblies (108) that each comprise:
at least one chamber unit (110) in which the biological material is receivable, the at least one chamber unit being defined in the substrate and extending between the top side and the bottom side;
an inlet (112) for receiving fluid;
an outlet (114) for allowing removal of fluid;
an inlet fluid channel (116) defined in the substrate and extending between the inlet and the at least one chamber unit;
an outlet fluid channel (118) defined in the substrate and extending between the at least one chamber unit and the outlet; and
a dam assembly for controlling fluid flow through the at least one chamber unit, the dam assembly comprising at least one of an inlet dam (136) and an outlet dam (132), the inlet dam being between the inlet fluid channel and the at least one chamber unit and upstanding towards the top side to an inlet threshold (138) arranged to permit fluid from the inlet fluid channel to flow thereover to be received in the at least one chamber unit, and the outlet dam being between the at least one chamber unit and the outlet fluid channel and upstanding towards the top side to an outlet threshold (134) arranged to permit fluid from the at least one chamber unit to flow thereover to be received in the outlet fluid channel.

2. The fluidic device (100) according to claim 1, wherein the at least one chamber unit (110) extends to an opening at the top side (104) of the substrate (102), and wherein the dam assembly comprises the inlet dam (136) and the outlet dam (132), the inlet dam and the outlet dam being arranged to retain fluid in the at least one chamber unit.

3. The fluidic device (100) according to claim 1 or claim 2, wherein the dam assembly comprises the inlet dam (136) and the outlet dam (132), and wherein the outlet threshold (134) is lower than the inlet threshold (138) so that fluid filling the at least one chamber unit (110) to reach the outlet threshold flows over the outlet threshold and into the outlet fluid channel (118).

4. The fluidic device (100) according to any one of claims 1 to 3, wherein a dual chamber unit (110) is included in one or more of said at least one chamber unit, said dual chamber unit comprising a first chamber (140) and a second chamber (142) fluidly connected to each other via a bridge (144) defined between the first chamber and the second chamber.

5. The fluidic device (100) according to claim 4, wherein:
the dam assembly comprises the inlet dam (136) and the outlet dam (132);
the inlet dam is between the inlet fluid channel (116) and the first chamber (140) so that fluid filling the inlet fluid channel to reach the inlet threshold (138) flows over the inlet threshold into the first chamber;
the outlet dam is between the second chamber (142) and the outlet fluid channel (118);
the fluidic device further comprises an intermediate dam (146) between the first and second chambers, the intermediate dam upstanding towards the top side to a bridge threshold at a base of the bridge (144) between the first chamber and the second chamber;
the bridge threshold is lower than the inlet threshold so that fluid filling the first chamber to reach the bridge threshold is permitted to flow over the bridge threshold into the second chamber; and
the outlet threshold (134) is lower than the bridge threshold so that fluid filling the second chamber to reach the outlet threshold flows over the outlet threshold into the outlet fluid channel.

6. The fluidic device (100) according to any one of claims 1 to 5, wherein each of the at least one chamber unit (110) comprises at least one supporting region (120) for supporting a biocompatible partition (122) so that the biocompatible partition separates an upper portion (124) from a lower portion (126) of the respective chamber unit.

7. The fluidic device (100) according to claim 6, wherein the upper and lower portions (124, 126) have different volumes relative to each other; and/or the at least one supporting region (120) is arranged to support the biocompatible partition closer to one of the top and bottom sides (104, 106) than the other of the top and bottom sides (106, 104).

8. The fluidic device (100) according to claim 6 or claim 7, comprising the biocompatible partition (122), wherein the biocompatible partition is attachable to the substrate (102) or is an integral part of the substrate.

9. The fluidic device (100) according to any one of claims 6 to 8, comprising:
an upper fluid flow system (128) defined in the substrate (102), the upper fluid flow system including the inlet fluid channel (116) and the outlet fluid channel (118); and
a lower fluid flow system (130) defined in the substrate and arranged to supply fluid to and/or permit fluid to leave said lower portion (126).

10. The fluidic device (100) according to any one of claims 1 to 9, wherein the at least one chamber unit (110) comprises a plurality of chamber units.

11. The fluidic device (100) according to claim 10, wherein the chamber units (110) are connected in parallel across the inlet fluid channel (116) and the outlet fluid channel (118).

12. The fluidic device (100) according to claim 10 or claim 11 as according to claim 9, wherein the plurality of chamber units (110) each comprise said upper portion (124) and said lower portion (126), and wherein the lower fluid flow system (130) comprises at least one fluid channel (131; 160, 162) that fluidly connects the lower portions to each other.

13. The fluidic device (100) according to any one of claims 1 to 12, comprising a plurality of said fluidic assemblies (108); optionally wherein the outlet thresholds (134) of at least some of the plurality of fluidic assemblies are at different heights relative to each other.

14. The fluidic device (100) according to any one of claims 1 to 13, wherein the inlet fluid channel (116) provides a resistance to flow therein that changes with distance along the inlet fluid channel away from the inlet (112); and/or wherein the outlet fluid channel (118) provides a resistance to flow therein that changes with distance along the outlet fluid channel away from the chamber unit(s) (110).

15. The fluidic device (100) according to any one of claims 1 to 14, comprising an upper cover for covering the top side (104) of the substrate (102) and/or a lower cover for covering the bottom side (106) of the substrate, optionally wherein one or both of the upper and lower covers is or are configured to enable access to the at least one chamber unit (110).

16. Use of the fluidic device (100) according to any one of claims 1 to 15 for testing biological material.

17. A drug testing method comprising:
providing biological material in the at least one chamber unit (110) of a fluidic device (100) according to any one of claims 1 to 15, optionally wherein said providing biological material in the at least one chamber unit comprises culturing cells in the at least one chamber unit; and
exposing the biological material to the drug to be tested.
